# EUROPEAN PATENT APPLICATION

(11) **EP 0 641 787 A1**
(43) Date of publication of application: **08.03.1995**
(21) Application number: 94810493.0
(22) Date of filing: 30.08.1994
(51) Int. Cl.: C07D 265/34, A61K 31/535

(54) **2H-naphth[2,3-b]-1,4-oxazine derivatives, their preparation and their use as serotonin 1D (5-HT10) receptor agonists**

(30) Priority: 03.09.1993 DE 4329776
(71) Applicant: SANDOZ LTD., CH-4002 Basel (CH); SANDOZ-PATENT-GMBH, D-79539 Lörrach (DE); SANDOZ-ERFINDUNGEN Verwaltungsgesellschaft m.b.H., A-1235 Wien (AT)
(72) Inventor: Nozulak, Joachim, D-79423 Heitersheim (DE)

(57) **Abstract**

Compounds of formula I 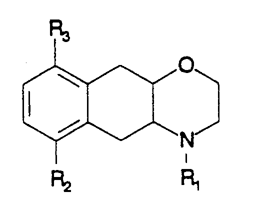
wherein

- R₁: is (C₁₋₄)alkyl, (C₃₋₆)alkenyl, whereby the double bond is not in α-position to the nitrogen atom, or (C₃₋₇)cycloalkyl(C₁₋₂)alkyl,
- R₂: is (C₁₋₄)alkoxy, (C₂₋₆)alkenyloxy or (C₃₋₇)cycloalkyl(C₁₋₂)alkoxy, and
- R₃: is (C₁₋₄)alkylcarbonyl, (C₁₋₄)alkyl, α-hydroxy(C₁₋₄)alkyl, (C₂₋₅)alkenyl, (C₁₋₄)alkoxy(C₁₋₄)alkyl or (C₃₋₇)cycloalkyl,

in free base or acid addition salt form, exhibit serotonin 1D (5-HT₁_{D})-receptor agonistic activity and are thus useful interalia in the treatment of migraine, depression, anxiety and pain.

## Description

The present invention relates to naphthoxazines, their production, their use as pharmaceuticals and pharmaceutical compositions containing them.

In particular the present invention provides compounds of formula I 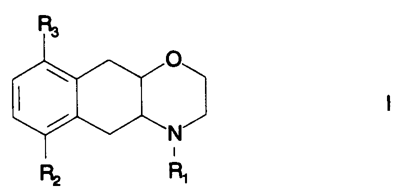
wherein
- R₁: is (C₁₋₄)alkyl, (C₃₋₆)alkenyl, whereby the double bond is not in α-position to the nitrogen atom, or (C₃₋₇)cycloalkyl(C₁₋₂)alkyl,
- R₂: is (C₁₋₄)alkoxy, (C₂₋₆)alkenyloxy or (C₃₋₇)cycloalkyl(C₁₋₂)alkoxy, and
- R₃: is (C₁₋₄)alkylcarbonyl, (C₁₋₄)alkyl, α-hydroxy(C₁₋₄)alkyl, (C₂₋₅)alkenyl, (C₁₋₄)alkoxy(C₁₋₄)alkyl or (C₃₋₇)cycloalkyl,

in free base or acid addition salt form, hereinafter referred to as new compounds.

The invention includes the racemic forms of the cis- and trans-isomers, which, on account of the asymmetrical carbon atoms, may be present in 4a- and 10a-position or within the substituent R₃, as well as the corresponding enantiomers.

The above-defined alkyl or alkoxy groups contained in the new compounds preferably contain 1 or 2 carbon atoms and in particular represent methyl or methoxy.

In one group of new compounds, R₁ is (C₁₋₄)alkyl.

In another group, R₂ is (C₁₋₄)alkoxy.

In a further group, R₃ is (C₁₋₄)alkylcarbonyl, (C₁₋₄)alkyl, α-hydroxy(C₁₋₄)alkyl, (C₂₋₅)alkenyl or (C₁₋₄)alkoxy(C₁₋₄)alkyl.

In still a further group, R₁ is methyl, ethyl or propyl, R₂ is methoxy and R₃ is acetyl, ethylcarbonyl, α-hydroxyethyl, α-hydroxypropyl, α-methoxyethyl, α-hydroxy-α-methylethyl, α-hydroxy-α-methylpropyl, ethenyl, α-methylethenyl, 1-propenyl, 1-methyl-1-propenyl, ethyl or propyl.

In accordance with the invention, the new compounds are obtained whereby
a) in order to produce compounds of formula Ia 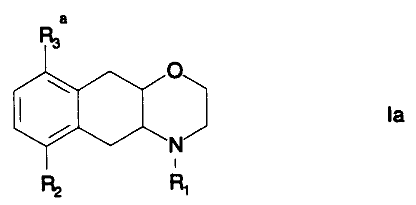 wherein R₁ and R₂ are as defined above, and R₃^{a} is (C₁₋₄)alkylcarbonyl, compounds of formula II 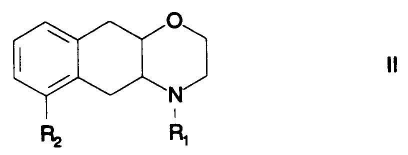 wherein R₁ and R₂ are as defined above, are acylated in 9-position, or
b) in order to produce compounds of formula Ib, 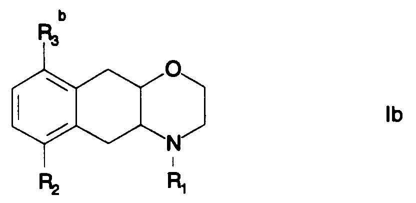 wherein R₁ and R₂ are as defined above and R₃^{b} is (C₁₋₄)alkyl, α-hydroxy(C₁₋₄)alkyl, (C₂₋₅)alkenyl, (C₁₋₄)alkoxy(C₁₋₄)alkyl or (C₃₋₇)cycloalkyl, compounds of formula II are alkylated in 9-position,

and, if desired, the compounds of formula I thus obtained are converted into their acid addition salts.

The acylation according to process a) may be effected in known manner, e.g. by means of a reaction with an acyl halide as described in example 1.

The alkylation according to progress b) may be effected in known manner, e.g. by means of a reaction with an alkyl halide. The process may also take place in two stages, whereby first of all the compounds of formula II are acylated as in process a), and are subsequently reduced. The 9-alkyl compounds are obtained e.g. by means of catalytic hydrogenation as described in example 13. In order to produce the 9-hydroxyalkyl derivatives, reduction is carried out e.g. with complex hydrides such as sodium borohydride or lithium aluminium hydride, as described in example 3, or the procedure of example 6 may be followed. In order to produce the 9-alkoxyalkyl derivatives, the process may also take place in three stages, whereby the 9-hydroxyalkyl derivatives are etherified (see example 5). The 9-alkenyl derivatives may similarly be produced from the 9-hydroxyalkyl derivatives by splitting off water (see example 8).

Working up of the reaction mixtures obtained according to the above processes and purification of the compounds of formula I thus obtained may be carried out in accordance to known procedures.

If desired, enantiomer separation may be effected by known methods, e.g. at the stage of the compounds of formula II or at a preliminary stage.

The compounds of formula I may be present in free form or in the form of their addition salts with acids. Acid addition salts may be produced in known manner from the free base forms, and vice versa. Such salts include for example the hydrochloride or the hydrogen malonate.

The starting compounds of formula II are known e.g. from US patent specification 4,656,167.

The compounds of formula I and their physiologically acceptable acid addition salts, hereinafter referred to as compounds according to the invention, have interesting pharmacological properties when tested on animals, and may therefore be used as medicaments.

The compounds according to the invention have binding affinity to serotonin-1D receptors, which was established according to the method of C. Weber et al., Naunyn-Schmiedeberg's Arch. Pharmacol. 337, 595 - 601 (1988). They are 5HT-1D receptor agonists, as may be seen from the following test:

Anterior cerebral arteries are excised from pig brains. Circular segments of 3-4 mm length are mounted between two L-shaped metal prongs and placed in temperature-controlled (37°C) baths consisting of a Krebs solution which is constantly gassed with 5% CO₂ in oxygen. Agonist-induced, vascular contractions are measured isometrically. In order to measure exclusively the serotonin-1D receptor mediated effects, ketanserin (10⁻⁷ M), which prevents contractions via serotonin-2 receptors, is added to the bath solution. The compounds according to the invention induce vascular contractions at concentrations between 10⁻¹¹ and 10⁻⁵ M.

As a result of this activity, the compounds according to the invention are useful to treat conditions associated with headaches, especially migraine, cluster headache, chronic paroxysmal hemicrania and headaches associated with vascular disorders, and to reduce the symptoms linked therewith. Furthermore they are useful as analgesics for the treatment of pain of various origin.

These 5HT-1D receptor agonists penetrate the central nervous system, as may be seen from their ability to compensate the reserpine-induced electric spiking activity of the pontine, geniculo-occipital region of the cat [method according to Zueger et al., Experientia 34, 637 - 639 (1978)].

As a result of the above activities, the compounds according to the invention are useful to treat Parkinson's disease, anxiety, depression, anorexia nervosa or bulimia, and also to treat obsessive, compulsive disorders.

The anxiolytic/antidepressant activity is preferred and can be confirmed in the following tests:

In the sleep/wake cycle of the long-term implanted rat [Vigouret et al., Pharmacology 16 Suppl. A 156 (1978)], the compounds according to the invention bring about a lengthening of the wake phases at doses of about 0.1 to 10 mg/kg p.o.

In a behavioral study using rhesus macaques and conducted as described by D. Maestripieri et al. in Developmental Psychobiology 24(8):571-581 (1992), the compounds according to the invention exhibit anxiolytic properties at doses of about 0.01 to 1 mg/kg p.o.

For the above-mentioned indications, the daily dosage ranges from about 1 to about 50 mg of a compound according to the invention, conveniently administered, for example, in divided doses up to four times a day.

The compounds according to the invention may be administered by any conventional route, in particular enterally, preferably orally, for example in the form of tablets or capsules, or parenterally, for example in the form of injectable solutions or suspensions.

In accordance with the foregoing, the present invention also provides a compound according to the invention, for use as a pharmaceutical, e.g. for the treatment of migraine, pain, depression and anxiety.

The present invention furthermore provides a pharmaceutical composition comprising a compound according to the invention in association with at least one pharmaceutical carrier or diluent. Such compositions may be manufactured in conventional manner. Unit dosage forms contain, for example, from about 0.25 to about 25 mg of a compound according to the invention.

Moreover the present invention provides the use of a compound according to the invention, for the manufacture of a medicament for the treatment of migraine, depression, anxiety or pain.

The following examples illustrate the invention. The temperatures are given in degrees Celsius and are uncorrected.

### EXAMPLE 1:

**(-)-(4aR,10aR)-9-acetyl-3,4,4a,5,10,10a-hexahydro-6-methoxy-4-methyl-2H-naphth[2,3-b]-1,4-oxazine**

23.30 g (0.10 mol) of (-)-(4aR,10aR)-3,4,4a,5,10,10a-hexahydro-6-methoxy-4-methyl-2H-naphth[2,3-b]-1,4-oxazine are dissolved in methylene chloride at 0°. 26.70 g (0.20 mols) of aluminium trichloride are added, and subsequently 7.85 g (0.10 mol) of acetyl chloride are added to the suspension in drops. The preparation is stirred for 4 hours at room temperature, and is subsequently mixed with a mixture of ice water and 2 N caustic soda solution. The methylene chloride phase is washed several times with ice water, dried and concentrated by evaporation. After crystallization from acetone / ether / petroleum ether, (-)-(4aR,10aR)-9-acetyl-3,4,4a,5,10,10a-hexahydro-6-methoxy-4-methyl-2H-naphth[2,3-b]-1,4-oxazine is obtained with the melting point of 134-135° and rotation value of [α]_{D}²⁰ = -226° (c = 0.5, methylene chloride), as the hydrochloride with the melting point of 265-266° (decomp.) [crystallizes from methanol/methylene chloride/acetone] and the rotation value of [α]_{D}²⁰ = -189° (c = 0.5, methanol).

The starting material may be produced as follows:
**a)** **(-)-(4aR,10aR)-3,4,4a,5,10,10a-hexahydro-6-methoxy-2H-naphth[2,3-b]-1,4-oxazine**
   39.00 g (0.178 mol) of (±)-trans-3,4,4a,5,10,10a-hexahydro-6-methoxy-2H-naphth[2,3-b]-1,4-oxazine (known e.g. from US patent specification 4.656.167) are dissolved in ethanol. A solution of 26.70 g (0.178 mols) of L-(+)-tartaric acid in water is added. The crystallizate which precipitates at 0° is filtered off by suction, washed with ice water and recrystallized from boiling water until reaching a constant optical rotation, as determined by measuring the rotation value of the free base. The base is subsequently released by adding 2 N caustic soda solution and methylene chloride. The methylene chloride phase is separated, dried and concentrated by evaporation. (-)-(4aR,10aR)-3,4,4a,5,10,10a-hexahydro-6-methoxy-2H-naphth[2,3-b]-1,4-oxazine is obtained with the rotation value of [α]_{D}²⁰ = -83.5° (c = 2, ethanol). The tartrate has a melting point of 244° (decomposition).
**b)** **(-)-(4aR,10aR)-3,4,4a,5,10,10a-hexahydro-6-methoxy-4-methyl-2H-naphth[2,3-b]-1,4-oxazine**
   17.50 g (80 mmol) of (-)-(4aR,10aR)-3,4,5a,5,10,10a-hexahydro-6-methoxy-2H-naphth[2,3-b]-1,4-oxazine are dissolved in methanol. 2.60 g of palladium on carbon (10%) is added, and then 94.70 g (ca. 1.2 mol) of formaldehyde (ca. 37% solution in water) are added to the reaction mixture. Hydrogenation is subsequently effected under normal pressure at 30° with hydrogen. After 4 hours, the reaction mixture is filtered over Hyflo and the solvent concentrated by evaporation. The residue is taken up in toluene, mixed with saturated sodium hydrogen carbonate solution and stirred. The solution is subsequently filtered and the toluene phase is separated. The organic phase is dried over magnesium sulphate and concentrated by evaporation. (-)-(4aR,10aR)-3,4,4a,5,10,10a-hexahydro-6-methoxy-4-methyl-2H-naphth[2,3-b]-1,4-oxazine is obtained with the rotation value of [α]_{D}²⁰ = -137.2° (c = 2, ethanol); 1H-NMR (CDCl3): d = 2.40 (s; 3 H, N-CH3), 3.83 (s; 3 H, O-CH3).

### EXAMPLE 2:

**(±)-(4aR,10aR)-9-(1'-oxopropyl)-3,4,4a,5,10,10a-hexahydro-6-methoxy-4-methyl-2H-naphth[2,3-b]-1,4-oxazine**

Preparation analogous to example 1.
M.p. of the hydrochloride: 272-274°.
M.p. of the base: 102-104°.

### EXAMPLE 3:

**(-)-(1'S,4aR,10aR)-3,4,4a,5,10,10a-hexahydro-9-(1'-hydroxyethyl)-6-methoxy-4-methyl-2H-naphth[2,3-b]-1,4-oxazine and (-)-(1'R,4aR,10aR)-3,4,4a,5,10,10a-hexahydro-9-(1'-hydroxyethyl)-6-methoxy-4-methyl-2H-naphth[2,3-b]-1,4-oxazine**

9.50 g (250 mmol) of lithium aluminium hydride are suspended in tetrahydrofuran. A solution of 13.80 g (50 mmol) of (-)-(4aR,10aR)-9-acetyl-3,4,4a,5,10,10a-hexahydro-6-methoxy-4-methyl-2H-naphth[2,3-b]-1,4-oxazine in tetrahydrofurane is added to the suspension in drops at room temperature, and the reaction mixture is allowed to boil under reflux for 1½ hours. Subsequently, the excess lithium aluminium hydride is broken down with water at -20°, and the suspension is mixed with methylene chloride. The methylene chloride extract is dried and concentrated by evaporation. After separation by column chromatography (silica gel, ethyl acetate/hexane, 1:1) and recrystallization from acetone, (-)-(1'S,4aR,10aR)-3,4,4a,5,10,10a-hexahydro-9-(1'-hydroxyethyl)-6-methoxy-4-methyl-2H-naphth[2,3-b]-1,4-oxazine is obtained with the melting point of 149°. The (-)-(1'S,4aR,10aR)-3,4,4a,5,10,10a-hexahydro-9-(1'-hydroxyethyl)-6-methoxy-4-methyl-2H-naphth[2,3-b]-1,4-oxazine hydrochloride has the melting point > 248° (decomposition) and the rotation value of [α]_{D}²⁰ = -115° (c = 0.55, dimethyl sulphoxide).

The second epimer obtained during chromatography is converted into the meso-hydrogen tartrate. (-)-(1'R,4aR,10aR)-3,4,4a,5,10,10a-hexahydro-9-(1'-hydroxyethyl)-6-methoxy-4-methyl-2H-naphth[2,3-b]-1,4-oxazine-meso-hydrogen-tartrate is obtained with the melting point 155-156° (crystallization from methanol/ethanol/acetone) and the rotation value of [α]_{D}²⁰ = -62° (c = 0.5, dimethyl sulphoxide).

### EXAMPLE 4:

**(±)-trans-3,4,4a,5,10,10a-hexahydro-9-(1'-hydroxypropyl)-6-methoxy-4-methyl-2H-naphth[2,3-b]-1,4-oxazine**

Preparation analogous to example 3.
M.p. of the hydrogen malonates: 140 - 141° and 168 - 170°
(diastereoisomers as regards position 1').

### EXAMPLE 5:

**(-)-(1'S,4aR,10aR)-3,4,4a,5,10,10a-hexahydro-9-(1'-methoxyethyl)-6-methoxy-4-methyl-2H-naphth[2,3-b]-1,4-oxazine and (-)-(1'R,4aR,10aR)-3,4,4a,5,10,10a-hexahydro-9-(1'-methoxyethyl)-6-methoxy-4-methyl-2H-naphth[2,3-b]-1,4-oxazine**

3.00 g (10.8 mmol) of (-)-(1'S,4aR,10aR)-3,4,4a,5,10,10a-hexahydro-9-(1'-hydroxyethyl)-6-methoxy-4-methyl-2H-naphth[2,3-b]-1,4-oxazine are dissolved in methylene chloride. To the solution are added 3.67 g (10.8 mmol) of tetrabutylammonium hydrogen sulphate and 0.67 ml (10.8 mmol) of methyl iodide. 30% caustic soda solution is subsequently added, and the reaction mixture is stirred intensely at room temperature. After one hour, a further 3.67 g (10.8 mmol) of tetrabutylammonium hydrogen sulphate and 0.67 ml (10.8 mmol) of methyl iodide are added. After a further 45 minutes, 1.84 g (5.4 mmol) of tetrabutylammonium hydrogen sulphate and 0.34 ml (5.4 mmol) of methyl iodide are again added. After a total of 2½ hours, the preparation is poured onto ice, the methylene chloride phase is separated, washed with water and dried. After purification by column chromatography (silica gel, ethyl acetate/hexane, 1:1), (-)-(1'S,4aR,10aR)-3,4,4a,5,10,10a-hexahydro-9-(1'-methoxyethyl)-6-methoxy-4-methyl-2H-naphth[2,3-b]-1,4-oxazine is obtained with the melting point 92 - 93° and the rotation value of [α]_{D}²⁰ = -188.8° (c = 0.52, methanol). With fumaric acid, the base forms the hydrogen fumarate with a melting point of 158 - 160° (crystallization from methanol/acetone/ether) and has the rotation value of [α]_{D}²⁰ = -138° (c = 0.52, methanol). The second epimer is etherified analogously to the above procedure. (-)-(1'R,4aR,10aR)-3,4,4a,5,10,10a-hexahydro-9-(1'-methoxyethyl)-6-methoxy-4-methyl-2H-naphth[2,3-b]-1,4-oxazine is obtained with the melting point 74 - 76° (crystallization from ether/petroleum ether) and the rotation value of [α]_{D}²⁰ = -36.8° (c = 0.53, methanol). With maleic acid, the base forms the hydrogen maleinate with the melting point 185 - 187° (crystallization from methanol / acetone / ether) and the rotation value of [α]_{D}²⁰ = -28.6° (c = 0.51, methanol). The hydrochloride melts at 204 - 205°

### EXAMPLE 6:

**(-)-(4aR,10aR)-3,4,4a,5,10,10a-hexahydro-9-(1'-hydroxy-1'-methylethyl)-6-methoxy-4-methyl-2H-naphth[2,3-b]-1,4-oxazine**

14.7 ml (23.5 mmol) of methyl lithium (1.6 M solution in ether) are added to absolute hexane at -20°. At the same temperature, a solution of 3.23 g (11.8 mmol) of (-)-(4aR,10aR)-9-acetyl-3,4,4a,5,10,10a-hexahydro-6-methoxy-4-methyl-2H-naphth[2,3-b]-1,4-oxazine in absolute ether is added in drops. The cooling bath is subsequently removed, and stirring is effected for 10 minutes. The reaction mixture is quenched at -5° with ammonium chloride solution, and poured into ethyl acetate. The organic phase is washed with water, dried and concentrated by evaporation. The residue is dissolved in acetone/methanol, and mixed with malonic acid. After evaporation of the solvent and recrystallization from methanol/acetone/ether, (-)-(4aR,10aR)-3,4,4a,5,10,10a-hexahydro-9-(1'-hydroxy-1'-methylethyl)-6-methoxy-4-methyl-2H-naphth[2,3-b]-1,4-oxazine-hydrogen-malonate is obtained with a melting point of 163 - 164° and the rotation value of [α]_{D}²⁰ = -67° (c = 0.5, methanol).

### EXAMPLE 7:

**(±)-trans-3,4,4a,5,10,10a-hexahydro-9-(1'-hydroxy-1'-methylpropyl)-6-methoxy-4-methyl-2H-naphth[2,3-b]-1,4-oxazine**

Preparation analogous to example 6.
M.p. of the hydrogen malonates: 158 - 160° and 155 - 156°.
M.p. of the bases: 180 - 181° and 158 - 160°.
(diastereoisomers as regards position 1')

### EXAMPLE 8:

**(±)-trans-9-ethenyl-3,4,4a,5,10,10a-hexahydro-6-methoxy-4-methyl-2H-naphth[2,3-b]-1,4-oxazine**

4.88 ml (32.5 mmol) of 4-toluenesulphonyl isocyanate are dissolved in toluene at 0°. A solution of 9.01 g (32.5 mmol) of (±)-3,4,4a,5,10,10a-hexahydro-9-(1-hydroxyethyl)-6-methoxy-4-methyl-2H-naphth[2,3-b]-1,4-oxazine (production as in example 3) in toluene is added to this in drops. The reaction mixture is heated under reflux for 24 hours, then cooled and mixed directly with ethyl acetate / 2N sulphuric acid / ice. The separated acetic ester phase is rejected, the aqueous phase is rendered basic with concentrated ammonia solution, and the product is extracted with acetic ester. The organic phase is dried and concentrated by evaporation. After recrystallization of the residue from methylene chloride / methanol / ether, (±)-trans-9-ethenyl-3,4,4a,5,10,10a-hexahydro-6-methoxy-4-methyl-2H-naphth[2,3-b]-1,4-oxazine is obtained with a melting point of 255 - 258°. After adding malonic acid in acetone and subsequently evaporating the solvent, the product is recrystallized from methanol / methylene chloride / ether to yield the (±)-trans-9-ethenyl-3,4,4a,5,10,10a-hexahydro-6-methoxy-4-methyl-2H-naphth[2,3-b]-1,4-oxazine-hydrogen-malonate with a melting point of 174 - 175°.

### EXAMPLE 9:

**(-)-4aR,10aR)-3,4,4a,5,10,10a-hexahydro-6-methoxy-4-methyl-9-(1'-methylethenyl)-2H-naphth[2,3-b]-1,4-oxazine**

Preparation analogous to example 8.
M.p. of the hydrochloride: 261 - 262°.
[α]_{D}²⁰ = -66° (c = 0.5, methanol).

### EXAMPLE 10:

**(-)-(4aR,10aR)-3,4,4a,5,10,10a-hexahydro-6-methoxy-9-(1'-methylethenyl)-4-n-propyl-2H-naphth[2,3-b]-1,4-oxazine**

Preparation analogous to example 8.
M.p. of the hydrochloride: 209 - 211°.
[α]_{D}²⁰ = -89.6° (c = 0.5, water).

### EXAMPLE 11:

**(±)-trans-3,4,4a,5,10,10a-hexahydro-6-methoxy-4-methyl-9-(1'-propenyl)-2H-naphth[2,3-b]-1,4-oxazine**

Preparation analogous to example 8.
M.p. of the hydrochloride: 212 - 214° (decomp.).

### EXAMPLE 12:

**(±)-trans-3,4,4a,5,10,10a-hexahydro-6-methoxy-4-methyl-9-(1'-methyl-1'-propenyl)-2H-naphth[2,3-b]-1,4-oxazine**

Preparation analogous to example 8.
M.p. of the hydrochloride: 224 - 226°.

### EXAMPLE 13:

**(-)-(4aR,10aR)-9-ethyl-3,4,4a,5,10,10a-hexahydro-6-methoxy-4-methyl-2H-naphth[2,3-b]-1,4-oxazine**

4.13 g (15 mmol) of (-)-(4aR,10aR)-9-acetyl-3,4,4a,5,10,10a-hexahydro-6-methoxy-4-methyl-2H-naphth[2,3-b]-1,4-oxazine are hydrogenated together with 2 g of platinum oxide in 130 ml of 3 N ethanolic hydrochloric acid in a Parr apparatus at a hydrogen pressure of 5 hPa (5 bar). After 16 hours, the catalyst is filtered off, washed with ethanol and the filtrate concentrated by evaporation. The residue is mixed with 2 N caustic soda solution / ice, and extracted several times with methylene chloride. The combined methylene chloride phases are dried and concentrated by evaporation. After recrystallization from acetone / petroleum ether, (-)-(4aR,10aR)-9-ethyl-3,4,4a,5,10,10a-hexahydro-6-methoxy-4-methyl-2H-naphth[2,3-b]-1,4-oxazine is obtained with the melting point 94 - 96° and the rotation value of [α]_{D}²⁰ = -143° (c = 0.5, acetone). After a reaction with malonic acid in acetone, (-)-(4aR,10aR)-9-ethyl-3,4,4a,5,10,10a-hexahydro-6-methoxy-4-methyl-2H-naphth[2,3-b]-1,4-oxazine-hydrogen-malonate is obtained with the melting point 86 - 88° (crystallization from acetone/ether) and the rotation value of [α]_{D}²⁰ = -80° (c = 0.5, acetone).

### EXAMPLE 14:

**(±)-trans-3,4,4a,5,10,10a-hexahydro-6-methoxy-4-methyl-9-n-propyl-2H-naphth[2,3-b]-1,4-oxazine**

Preparation analogous to example 13.
M.p. of the hydrogen malonate: 123 - 125°.

## Claims

1. A compound of formula I 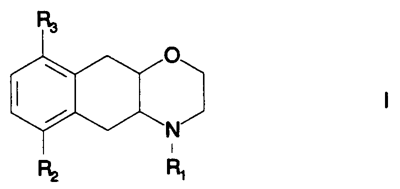 wherein
R₁ is (C₁₋₄)alkyl, (C₃₋₆)alkenyl, whereby the double bond is not in α-position to the nitrogen atom, or (C₃₋₇)cycloalkyl(C₁₋₂)alkyl,
R₂ is (C₁₋₄)alkoxy, (C₂₋₆)alkenyloxy or (C₃₋₇)cycloalkyl(C₁₋₂)alkoxy, and
R₃ is (C₁₋₄)alkylcarbonyl, (C₁₋₄)alkyl, α-hydroxy(C₁₋₄)alkyl, (C₂₋₅)alkenyl, (C₁₋₄)alkoxy(C₁₋₄)alkyl or (C₃₋₇)cycloalkyl,
in free base or acid addition salt form.

2. The (-)-(1'R, 4aR, 10aR)-3,4,4a,5,10,10a-hexahydro-9-(1'-methoxyethyl)-6-methoxy-4-methyl-2H-naphth[2,3-b]-1,4-oxazine in free base or acid addition salt form.

3. A compound of claim 1 selected from (-)-(4aR,10aR)-9-acetyl-3,4,4a,5,10,10a-hexahydro-6-methoxy-4-methyl-2H-naphth[2,3-b]-1,4-oxazine, (±)-(4aR,10aR)-9-(1'-oxopropyl)-3,4,4a,5,10,10a-hexahydro-6-methoxy-4-methyl-2H-naphth[2,3-b]-1,4-oxazine, (-)-(1'S,4aR,10aR)-3,4,4a,5,10,10a-hexahydro-9-(1'-hydroxyethyl)-6-methoxy-4-methyl-2H-naphth[2,3-b]-1,4-oxazine, (-)-1'R,4aR,10aR)-3,4,4a,5,10,10a-hexahydro-9-(1'-hydroxyethyl)-6-methoxy-4-methyl-2H-naphth[2,3-b]-1,4-oxazine, (±)-trans-3,4,4a,5,10,10a-hexahydro-9-(1'-hydroxypropyl)-6-methoxy-4-methyl-2H-naphth[2,3-b]-1,4-oxazine, (-)-(1'S, 4aR,10aR)-3,4,4a,5,10,10a-hexahydro-9-(1'-methoxyethyl)-6-methoxy-4-methyl-2H-naphth[2,3-b]-1,4-oxazine, (-)-(4aR,10aR)-3,4,4a,5,10,10a-hexahydro-9-(1'-hydroxy-1'-methylethyl)-6-methoxy-4-methyl-2H-naphth[2,3-b]-1,4-oxazine, (±)-trans-3,4,4a,5,10,10a-hexahydro-9-(1'-hydroxy-1'-methylpropyl)-6-methoxy-4-methyl-2H-naphth[2,3-b]-1,4-oxazine, (±)-trans-9-ethenyl-3,4,4a,5,10,10a-hexahydro-6-methoxy-4-methyl-2H-naphth[2,3-b]-1,4-oxazine, (-)-(4aR,10aR)-3,4,4a,5,10,10a-hexahydro-6-methoxy-4-methyl-9-(1'-methylethenyl)-2H-naphth[2,3-b]-1,4-oxazine, (-)-(4aR,10aR)-3,4,4a,5,10,10a-hexahydro-6-methoxy-9-(1'-methylethenyl)-4-n-propyl-2H-naphth[2,3-b]-1,4-oxazine, (±)-trans-3,4,4a,5,10,10a-hexahydro-6-methoxy-4-methyl-9-(1'-propenyl)-2H-naphth[2,3-b]-1,4-oxazine, (±)-trans-3,4,4a,5,10,10a-hexahydro-6-methoxy-4-methyl-9-(1'methyl-1'-propenyl)-2H-naphth[2,3-b]-1,4-oxazine, (-)-(4aR,10aR)-9-ethyl-3,4,4a,5,10,10a-hexahydro-6-methoxy-4-methyl-2H-naphth[2,3-b]-1,4-oxazine and (±)-trans-3,4,4a,5,10,10a-hexahydro-6-methoxy-4-methyl-9-n-propyl-2H-naphth[2,3-b]-1,4-oxazine, in free base or acid addition salt form.

4. A compound of any one of claims 1 to 3 in free base or physiologically acceptable acid addition salt form, for use as a pharmaceutical.

5. A compound of any one of claims 1 to 3 in free base or physiologically acceptable acid addition salt form, for use in the treatment of migraine or pain.

6. A compound of any one of claims 1 to 3 in free base or physiologically acceptable acid addition salt form, for use in the treatment of depression.

7. A compound of any one of claims 1 to 3 in free base or physiologically acceptable acid addition salt form, for use in the treatment of anxiety.

8. A pharmaceutical composition comprising a compound of any one of claims 1 to 3 in free base or physiologically acceptable acid addition salt form, in association with a pharmaceutical carrier or diluent.

9. The use of a compound of any one of claims 1 to 3 in free base or physiologically acceptable acid addition salt form, for the manufacture of a medicament for the treatment of migraine, depression, anxiety or pain.

10. A process for the production of compounds of formula I defined in claim 1, whereby
a) in order to produce compounds of formula Ia 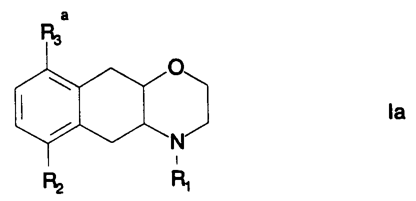 wherein R₁ and R₂ are as defined in claim 1 and R₃^{a} is (C₁₋₄)alkylcarbonyl, compounds of formula II 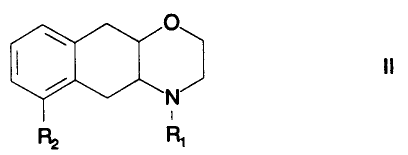 wherein R₁ and R₂ are as defined in claim 1, are acylated in 9-position, or
b) in order to produce compounds of formula Ib, 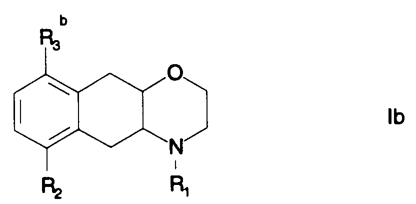 wherein
R₁ and R₂ are as defined in claim 1 and R₃^{b} is (C₁₋₄)alkyl, α-hydroxy(C₁₋₄)alkyl, (C₂₋₅)alkenyl, (C₁₋₄)alkoxy(C₁₋₄)alkyl or (C₃₋₇)cycloalkyl, compounds of formula II are alkylated in 9-position,
and, if desired, the compounds of formula I thus obtained are converted into their acid addition salts.
